# EUROPEAN PATENT SPECIFICATION

(11) **EP 3 801 408 B1**
(45) Date of publication and mention of the grant of the patent: **22.12.2021**
(21) Application number: 19726682.8
(22) Date of filing: 24.05.2019
(51) Int. Cl.: A61F 5/01

(54) **FORCE ANCHORING DEVICE**
KRAFTEINLEITUNGSVORRICHTUNG
DISPOSITIF D'ANCRAGE DE FORCE

(30) Priority: 01.06.2018 SE 1850670
(43) Date of publication of application: 14.04.2021
(73) Proprietor: Bioservo Technologies Aktiebolag, 164 40 Kista (SE)
(72) Inventor: EWALDSSON, Martin, Oskar, Gustaf, 193 40 Sigtuna (SE); INGVAST, Johan, 184 42 Åkersberga (SE); ASCARD, Martin, Olof, Hjalmar, 118 57 Stockholm (SE)
(74) Representative: Noréns Patentbyrå AB
(86) International application number: PCT/EP2019/063531
(87) International publication number: WO 2019/228933

(56) References cited:
- WO-A1-2013/006178
- US-A- 4 167 044
- US-A- 6 120 472
- US-A1- 2015 190 246

## Description

The present invention concerns a tendon actuated hand enhancer having a force anchoring device.

A problem with previously known tendon actuated hand enhancers, for example a grip enhancer according to WO 2008/027002 A1, is how to anchor the forces brought to the artificial tendons. Commonly, a soft cuff around the wrist is provided at which the anchoring has been carried out. The cuff needs to fit tightly to the wrist in order not to be drawn distally up on the hand. This may influence the carpal tunnel in the body in a negative way. Also, the hand will tend to flex downwards the arm when force is applied at at least one artificial tendon. It is also previously known to provide a stiff portion at a position corresponding to the palm of a hand of a user having anchoring in order to overcome the flexing of the hand when a force is applied at at least one artificial tendon.

US 2015/190246 discloses several embodiments of a tendon actuated hand enhancer comprising at least one artificial tendon, a pulling means for pulling the at least one artificial tendon and a force anchoring device, wherein at least one pulling point for said at least one artificial tendon is provided at the force anchoring device. In one embodiment the force anchoring device is a rigid wrist frame and in another embodiment the force anchoring device is a plate. US 2015/190246 fails to disclose that said force anchoring device comprises a rigid band circumventing more than half of a circumference of a wearer's hand in use, but being bendable in the circumferential direction, and that the force anchoring device is provided at the tendon actuated hand enhancer at a position corresponding to carpal and proximal portion of metacarpal bones of a user's hand when the hand enhancer is in use.

An aim of the present invention is to relieve as much as possible the problems with previously known art and provide comfortable, quickly responding tendon actuated hand enhancers by means of the novel force anchoring device.

According to an aspect of the present invention a tendon actuated hand enhancer having a force anchoring device is provided, which force anchoring device comprises a rigid band circumventing more than half of a circumference but is bendable in the circumferential direction. At least one pulling point for at least one artificial tendon is provided at the force anchoring device, and the force anchoring device is provided at the hand enhancer at a position corresponding to carpal bones and proximal portion of meta carpal bones of a user's hand when the hand enhancer is in use. A pulling means for pulling the at least one artificial tendon is connected to the hand enhancer.

By means of the tendon actuated hand enhancer having a force anchoring device a fixed anchoring of the forces is possible which provides a quick actuation of the at least one artificial tendons in the hand enhancer at the same time as the force anchoring device will rest on the area of the wearer's hand where the carpal and proximal portion of the meta carpal bones are situated. The rigidness of the force anchoring device provides the possibility of a fixed anchoring as it will not deform as previously known soft cuffs as well as not gliding up over the hand limiting the possibility to move at least the thumb.

According to an embodiment of the present invention the band has a slightly conical shape. A larger end of the conical band faces an end of the tendon actuated hand enhancer having at least one finger portion. Preferably, the band is preformed to correspond to the shape of a wearer's lower portion of the hand. This will further enhance the comfort for the wearer.

According to an embodiment a recess in the band is present in a position corresponding to a wearer's thumb in use. By means of this recess the wearer's thumb is free to move in any direction.

According to an embodiment the band is connectable by means of a connection to circumvent a wearer's lower portion of the hand at the position of the carpal and a proximal portion of the meta carpal bones. Preferably the connection is rigid but bendable in the circumferential direction. It is also preferred that the connection comprises a latch and release means.

According to an embodiment the at least one pulling point is provided on the ventral side of the band. According to an embodiment the band has fixing points for fixing a force balancing material on the dorsal side of the band.

### Short description of the drawings

The present invention will now be described under referral to the accompanying drawings, in which:
Fig. 1a shows an embodiment of a tendon actuated hand enhancer having a force anchoring device depicted in broken lines since it is embedded in the tendon actuated hand enhancer,
Fig. 1b shows an embodiment of a tendon actuated hand enhancer having a force anchoring device where the outer layer is removed to show parts within the hand enhancer,
Fig. 2a, b, c and d show different embodiments of a force anchoring device in a perspective view,
Fig. 3a and b show two embodiments of a force anchoring device with a rigid connection,
Fig. 3c shows an embodiment of a force anchoring device with a soft connection,
Fig. 4a shows an embodiment of a force anchoring device provided in a tendon actuated hand enhancer without the outer layer, and
Fig. 4b shows an embodiment of a tendon actuated hand enhancer having tendons at the dorsal side.

### Detailed description of embodiments of the invention

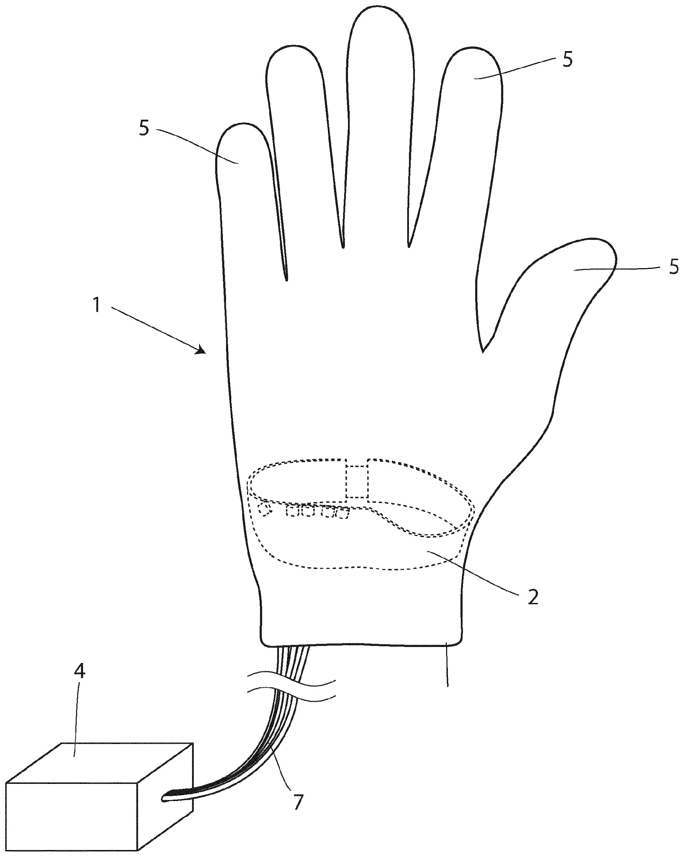

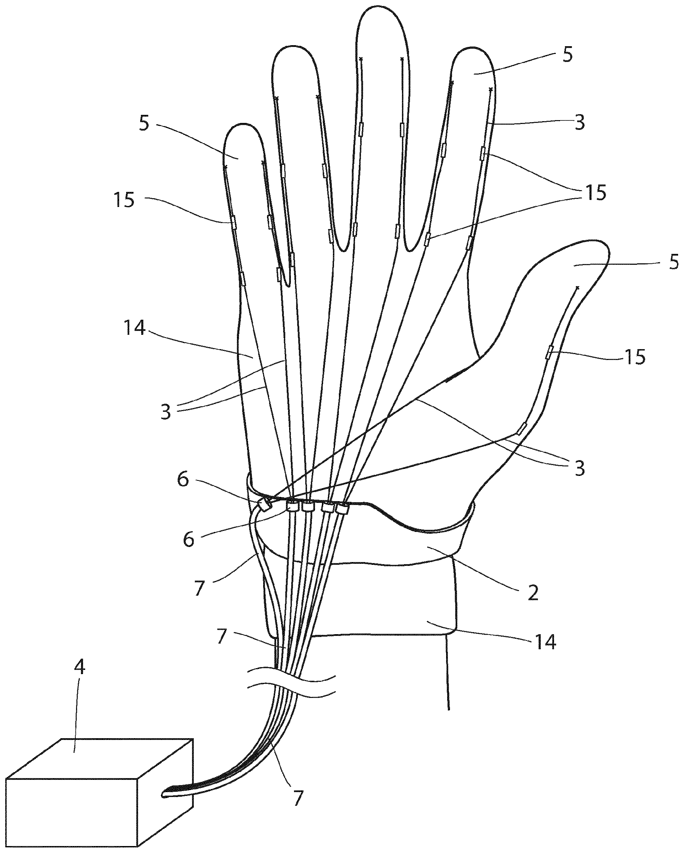

An embodiment of a tendon actuated hand enhancer 1 having a force anchoring device 2 is shown in Fig. 1a. In this embodiment an optional outer layer (for example a glove) is used and broken lines shows the force anchoring device so that it can be noticed although it does not need to be visible for a wearer when using the hand enhancer. In order to explain what a tendon actuated hand enhancer is, it is shown without an outer layer in Fig. 1b. At least one artificial tendon 3 runs between a pulling means 4 to at least one finger portion 5 of a force balancing material 14, such as an inner layer 14 (a thumb is also a finger). The artificial tendons 3 are attached at certain points or provided in, on or under the force balancing material 14. In the shown embodiment short ducts 15 attach the artificial tendons 3 to the inner layer 14 and the inner layer 14 is attached to the force anchoring device 2 at its inner side and protrudes towards the wrist. In some embodiments the artificial tendons 3 are attached on the inside of the outer layer. The force brought to the at least one artificial tendon 3 by the pulling means 4 is anchored in at least one pulling point 6 in the force anchoring device 2.

In case the pulling means 4 is not positioned at the pulling point 6 it is possible to use, for example, at least one Bowden cable 7 between the pulling means 6 and the at least one pulling point 6. Preferably, one Bowden cable is used for each artificial tendon 3 or each pair of artificial tendons 3 running to a specific finger portion 5. Also, one pulling point 6 per each artificial tendon 3 or each pair of artificial tendons 3 running to a specific finger portion 5 is provided. Preferably, sensors are located at at least the fingertips of the finger portions (not shown).

In the shown case 5 pairs of artificial tendons 3 run through a Bowden cable 7 each, which ends at a pulling point 6 each. At the pulling point 6 the force from the pulling means 4 is working, transmitted via the Bowden cable 7, on the artificial tendons 3. In the shown case all five finger portions 5 are present in the tendon actuated hand enhancer. It is also conceivable to have less finger portions 5, such as three, preferably for a thumb and two fingers.

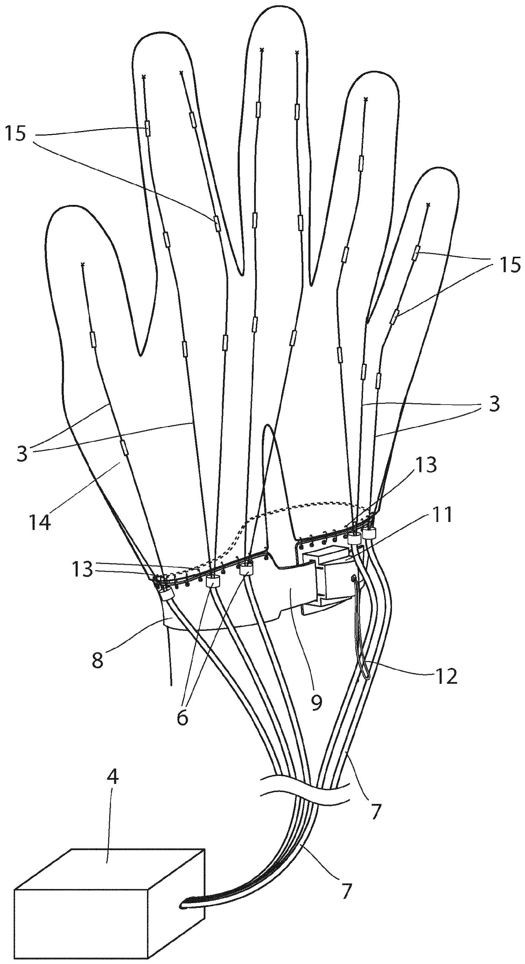

The tendon actuated hand enhancer 1 enhances for example the grip of a wearer's hand. It may also enhance the movement of the wearer's fingers to a straight position, for example, see Fig. 4b. The overall function of a tendon actuated hand enhance is to enhance the function and movement of a wearer's hand.

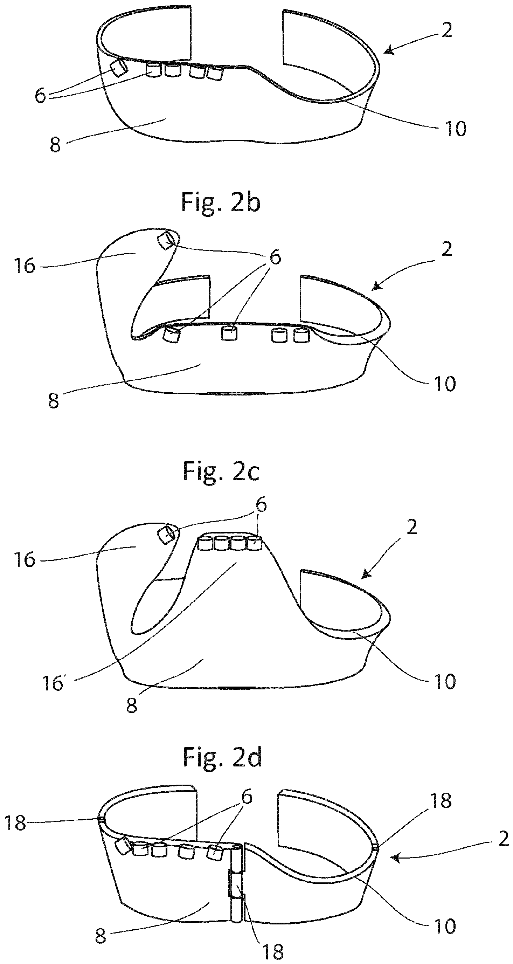

The force anchoring device 2 provides the novel and inventive feature of the present tendon actuated hand enhancer. In Fig. 2a a force anchoring device 2 is shown comprising a band 8 having a slightly conical shape circumventing more than half of a circumference of a wearer's hand. The band 8 is rigid but bendable, preferably in an elastic way, in the circumferential direction, i.e. bendable to and from a centre axis of the circumference. In this way it is possible to enlarge the circumference in order to let a hand of a wearer through and then it may spring back to an original shape. Preferably the band 8 is rigid in its surface plane. It is also conceivable to use links attached with joints 18 to provide the rigidness along the surface plane but be able to variate the circumference, which is shown in Fig. 2d. The bendability provides the possibility to be suitable for different sizes of hands of wearers.

As can be seen in Figs. 1a and 1b, the force anchoring device 2 has its larger end of the slightly conical shape arranged towards the at least one finger portion 5 of the hand enhancer 1. In this way it will rest comfortably at the proximal portion of the wearer's hand in a position corresponding to the carpal and proximal portion of the meta carpal bones in the wearer's hand. Due to the conical shape the force will be spread out over the surface of the force anchoring device 2. If the device would have been cylindrical all force would have been concentrated to the rim of the cylinder resting against the proximal portion of the wearer's hand, which would have been uncomfortable for the wearer.

At least one pulling point 6 is provided, preferably on the outside of the band 8, and preferably on the ventral side of the force anchoring device 2. Obviously, it is possible to arrange at least one pulling point 6 at the dorsal side if desired. Preferably, the shape of the band 8 corresponds as much as possible with a wearer's proximal end of the hand, i.e. at the position of the carpal and proximal portion of the meta carpal bones. In order not to delimit the possibility to move the thumb freely a recess 10 is provided at a position of the band 8 corresponding to the wearer's thumb or a thumb portion 5 of the hand enhancer 1.

In Figs. 2b and c the force anchoring device 12 is provided with protrusions 16, 16' where the pulling points 6 are provided. In Fig. 2a the protrusion 16 provides a pulling point 6 for the artificial tendons for the thumb. In Fig. 2c also the rest of the pulling points are provided at the protrusion 16'.

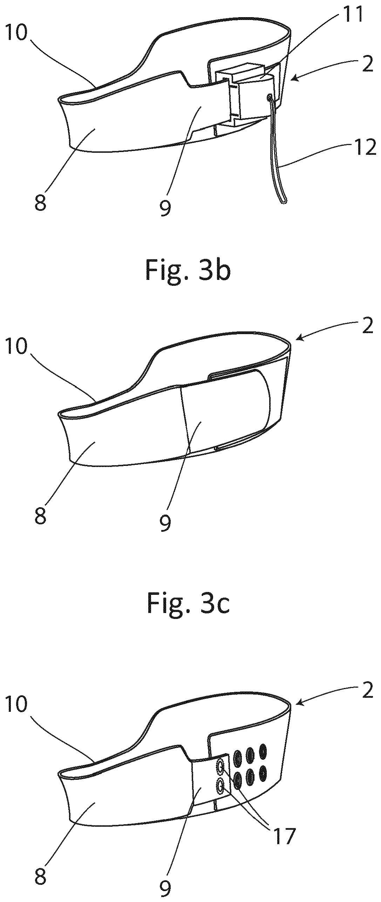

In Fig. 3a, b and c a connection 9 is shown between the two ends of the band 8. The connection 9 comprises a latch and release means 11 so that the force anchoring device 2 may be fixed into a suitable circumferential size for the wearer during use. This is preferably when the force anchoring device 2 tightly follows the proximal portion of the wearer's hand at a position corresponding to the carpal and proximal portion of the meta carpal bones but without squeezing the wearer. Thus, neither the carpal tunnel will be affected nor the movability of the wearer's hand. After use the wearer release the connection by means of the latch and release means 11 so that the circumference may be increased, and the hand of the wearer can be withdrawn through the force anchoring device 2 from the tendon actuated hand enhancer 1. Preferably, the latch and release means 11 is reachable from the outside of the tendon actuated hand enhancer 1. It may even be equipped with a quick release portion 12, such as a short strap.

In the shown embodiment of Fig. 3c the connection 9 is non-rigid, for example of a textile band. With this solution a certain warping deformation may occur in the force anchoring device 2. In Fig. 3c buttons 17 are used for the latch and release means 11. Obviously, other types of releasable attachments may be used, such as hook and loop attachment. In case less warping deformation is desired a rigid connection 9 should be used, as shown in the embodiment of Fig. 3a and b. In the embodiment of Fig. 3a the connection 9 is a portion of the band 8, i.e. an elongation of the band 8 which reaches a latch and release means 11 at the other end of the band 8.

The more rigid the force anchoring device 2 is the quicker response may be achieved in the tendon actuated hand enhancer 1. This is due to that when the hand enhancer 1 is applying force in the artificial tendons 3, i.e. the pulling means 4 will pull the artificial tendons 3 and build up force at the pulling point 6, which will be forced in the direction of the artificial tendon 3. The force anchoring device 2 will hold the pulling point 6 back. Force from the artificial tendons 3 will try to make the force anchoring device deflect. If a deflection of the force anchoring device according to previously known art occurs, it has to be compensated by extra pulling by the pulling means 4. This costs energy and requires longer stroke of the pulling means. The extra pulling also takes some time to perform, hence it reduces the responsiveness of the system. Thus, a force anchoring device that does not deflect much under force is desired.

It is also conceivable to provide a softer material, such as a foam polymer, textile or a nonwoven material, on the inside of the band 8 in order to enhance the comfort for the wearer. In Fig. 3b the connection 9 is rigid and fixedly attached to one end of the band 8 and connects with the other end of the band 8 by means a hook and loop means, such as Velcro, as the latch and release means 11.

In Fig. 3a an embodiment of connection 9 is shown having inclined steps (not shown) in the connection 9 and the elongated portion goes through a latch and release means 11 having a protrusion (not shown) engaging in a step to latch the connection 9. The protrusion is spring biased towards the steps but as the steps are inclined the protrusion will be pushed away along the inclination of a step during a tightening of the circumference and the protrusion will latch behind the step in the opposite direction. In orderto release the protrusion from a step when an enlargement of the circumference is desired the protrusion will be lifted, for example by means of a quick release portion 12, in the shown case a strap.

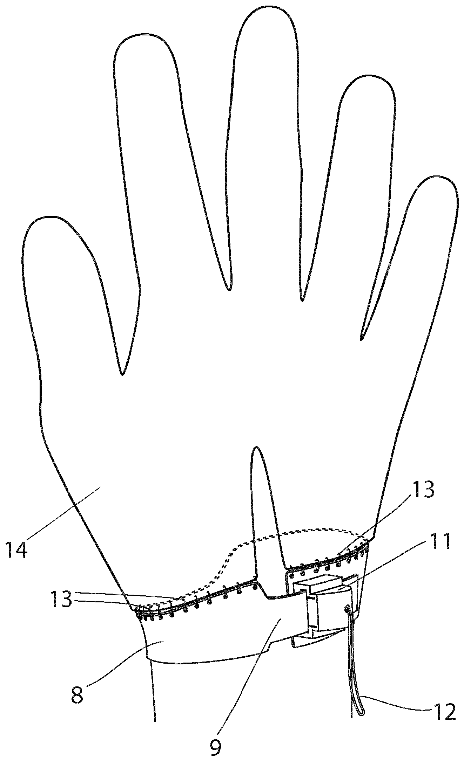

In Fig. 4a it is shown an embodiment of the tendon actuated hand enhancer 1 in a view without the outer layer and from a dorsal side of the hand enhancer 1. In the force anchoring device 2 there is at least one fixing point 13 for fixing a force balancing material 14, for example an inner layer as is shown in this embodiment, preferably on the dorsal side of the band 8 if the at least one pulling point 6 is provided on the ventral side of the band 8. In the shown embodiment the fixing points 13 are in the form of holes in the band 8 close to its larger rim and the inner layer 14 is sewn to the band with a thread going through the holes 13 and the inner layer 14. It is also conceivable to arrange at least one hook at the band 8 or other means for fixing the force balancing material 14 to the force anchoring device 2. Obviously, if the at least one pulling point 6 is provided on the dorsal side of the band 8, as shown in Fig. 4b, it is desired to have at least one fixing point (not shown) on the ventral side of the band 8.

In the shown embodiments of a tendon actuated hand enhancer 1 the artificial tendons 3 are provided on an inner layer 14 although it is also possible to provide them on the inside of the outer layer.

The force anchoring device may be produced by a variety of materials. For example, polymers, such as polyamide or polyethene, or hard elastomers, such as polyurethane or natural rubber. In case the band 8 is linked it is also possible to use even more rigid materials (on their own or in combination with the examples above), such as metals or composites armed with glass or carbon.

## Claims

1. A tendon actuated hand enhancer (1) comprising at least one artificial tendon (3), a pulling means for pulling the at least one artificial tendon (3) and a force anchoring device (2), wherein at least one pulling point (6) for said at least one artificial tendon (3) is provided at said force anchoring device (2), **characterised in that** said force anchoring device (2) comprises a rigid band (8) circumventing more than half of a circumference of a wearer's hand in use, but being bendable in the circumferential direction, and **in that** the force anchoring device (2) is provided at the tendon actuated hand enhancer (1) at a position corresponding to carpal and proximal portion of metacarpal bones of a user's hand when the hand enhancer (1) is in use.

2. A tendon actuated hand enhancer (1) having a force anchoring device (2) according to claim 1, wherein the band (8) has a slightly conical shape and a larger end of the conical band (8) faces an end of the tendon actuated hand enhancer (1) having at least one finger portion (5).

3. A tendon actuated hand enhancer (1) having a force anchoring device (2) according to claim 1 or 2, wherein the band (8) is preformed to correspond to the shape of a wearer's lower portion of the hand.

4. A tendon actuated hand enhancer (1) having a force anchoring device (2) according to claim 1, 2 or 3, wherein a recess (10) in the band (8) is present in a position corresponding to a wearer's thumb.

5. A tendon actuated hand enhancer (1) having a force anchoring device (2) according to any one of the preceding claims, wherein the band (8) is connectable by means of a connection (9) to circumvent a wearer's lower portion of the hand at the position of the carpal and the proximal portion of the meta carpal bones.

6. A tendon actuated hand enhancer (1) having a force anchoring device (2) according to claim 5, wherein the connection (9) is rigid but bendable in the circumferential direction.

7. A tendon actuated hand enhancer (1) having a force anchoring device (2) according to claim 5 or 6, wherein the connection (9) comprises a latch and release means (11).

8. A tendon actuated hand enhancer (1) having a force anchoring device (2) according to any one of the preceding claims, wherein the at least one pulling point (6) is provided on the ventral side of the band (8).

9. A tendon actuated hand enhancer (1) having a force anchoring device (2) according to any one of the preceding claims, wherein the band (8) has fixing points (13) for fixing a force balancing material (14) on the dorsal side of the band (8).

## Patentansprüche

1. Ein sehnenbetätigter Handverstärker (1), umfassend mindestens eine künstliche Sehne (3), ein Zugmittel zum Ziehen der mindestens einen künstlichen Sehne (3) und eine Kraftverankerungsvorrichtung (2), wobei mindestens ein Zugpunkt (6) für die mindestens eine künstliche Sehne (3) an der Kraftverankerungsvorrichtung (2) vorgesehen ist, **dadurch gekennzeichnet, dass** die Kraftverankerungsvorrichtung (2) ein starres Band (8) umfasst, das im Gebrauch mehr als die Hälfte des Umfangs der Hand eines Trägers umgibt, jedoch in der Umfangsrichtung biegbar ist, und dass die Kraftverankerungsvorrichtung (2) an dem sehnenbetätigten Handverstärker (1) an einer Position vorgesehen ist, die dem Handwurzelknochen und dem proximalen Abschnitt des Mittelhandknochens der Hand eines Benutzers entspricht, wenn der Handverstärker (1) im Gebrauch ist.

2. Ein sehnenbetätigter Handverstärker (1) mit einer Kraftverankerungsvorrichtung (2) nach Anspruch 1, wobei das Band (8) eine leicht konische Form aufweist und ein größeres Ende des konischen Bandes (8) einem Ende des sehnenbetätigten Handverstärkers (1) mit mindestens einem Fingerabschnitt (5) zugewandt ist.

3. Ein sehnenbetätigter Handverstärker (1) mit einer Kraftverankerungsvorrichtung (2) nach Anspruch 1 oder 2, wobei das Band (8) so vorgeformt ist, dass es der Form des unteren Teils der Hand eines Trägers entspricht.

4. Ein sehnenbetätigter Handverstärker (1) mit einer Kraftverankerungsvorrichtung (2) nach Anspruch 1, 2 oder 3, wobei eine Aussparung (10) im Band (8) in einer dem Daumen des Trägers entsprechenden Position vorhanden ist.

5. Ein sehnenbetätigter Handverstärker (1) mit einer Kraftverankerungsvorrichtung (2) nach einem der vorhergehenden Ansprüche, wobei das Band (8) mittels einer Verbindung (9) verbunden werden kann, um den unteren Teil der Hand eines Trägers an der Position des Handwurzelknochens und des proximalen Teils des Mittelhandknochens zu umgehen.

6. Ein sehnenbetätigter Handverstärker (1) mit einer Kraftverankerungsvorrichtung (2) nach Anspruch 5, wobei die Verbindung (9) starr, aber in Umfangsrichtung biegbar ist.

7. Ein sehnenbetätigter Handverstärker (1) mit einer Kraftverankerungsvorrichtung (2) nach Anspruch 5 oder 6, wobei die Verbindung (9) eine Verriegelungs- und Löseeinrichtung (11) umfasst.

8. Ein sehnenbetätigter Handverstärker (1) mit einer Kraftverankerungsvorrichtung (2) nach einem der vorhergehenden Ansprüche, wobei der mindestens eine Zugpunkt (6) auf der Ventralseite des Bandes (8) vorgesehen ist.

9. Ein sehnenbetätigter Handverstärker (1) mit einer Kraftverankerungsvorrichtung (2) nach einem der vorhergehenden Ansprüche, wobei das Band (8) Befestigungspunkte (13) zur Befestigung eines Kraftausgleichsmaterials (14) auf der Dorsalseite des Bandes (8) aufweist.

## Revendications

1. Dispositif d'amélioration de la main actionné par tendon (1) comprend au moins un tendon artificiel (3), un moyen de traction pour tirer le au moins un tendon artificiel (3) et un dispositif d'ancrage de force (2), dans lequel au moins un point de traction (6) pour ledit au moins un tendon artificiel (3) est prévu au niveau dudit dispositif d'ancrage de force (2), **caractérisé en ce que** ledit dispositif d'ancrage de force (2) comprend une bande rigide (8) contournant plus de la moitié d'une circonférence de la main d'un utilisateur à l'usage, mais n'étant pas pliable dans la direction circonférentielle, et **en ce que** le dispositif d'ancrage de force (2) est prévu au niveau du dispositif d'amélioration de la main actionné par tendon (1) dans une position correspondant à la partie carpienne et proximale des os métacarpiens de la main d'un utilisateur lorsque le dispositif d'amélioration de la main (1) est utilisé.

2. Dispositif d'amélioration de la main actionné par tendon (1) ayant un dispositif d'ancrage de force (2) selon la revendication 1, dans lequel la bande (8) a une forme légèrement conique et une plus grande extrémité de la bande conique (8) fait face à une extrémité du dispositif d'amélioration de la main actionné par tendon (1) ayant au moins une partie de doigt (5).

3. Dispositif d'amélioration de la main actionné par tendon (1) ayant un dispositif d'ancrage de force (2) selon la revendication 1 ou 2, dans lequel la bande (8) est préformée pour correspondre à la forme d'une partie inférieure de la main d'un utilisateur.

4. Dispositif d'amélioration de la main actionné par tendon (1) ayant un dispositif d'ancrage de force (2) selon la revendication 1, 2 ou 3, dans lequel un évidement (10) dans la bande (8) est présent dans une position correspondant au pouce d'un utilisateur.

5. Dispositif d'amélioration de la main actionné par tendon (1) ayant un dispositif d'ancrage de force (2) selon l'une quelconque des revendications précédentes, dans lequel la bande (8) peut être raccordée au moyen d'un raccordement (9) pour contourner la partie inférieure de la main d'un utilisateur dans la position de la partie carpienne et proximale des os métacarpiens.

6. Dispositif d'amélioration de la main actionné par tendon (1) ayant un dispositif d'ancrage de force (2) selon la revendication 5, dans lequel le raccordement (9) est rigide mais pliable dans la direction circonférentielle.

7. Dispositif d'amélioration de la main actionné par tendon (1) ayant un dispositif d'ancrage de force (2) selon la revendication 5 ou 6, dans lequel le raccordement (9) comprend un moyen de verrouillage et de libération (11).

8. Dispositif d'amélioration de la main actionné par tendon (1) ayant un dispositif d'ancrage de force (2) selon l'une quelconque des revendications précédentes, dans lequel le au moins un point de traction (6) est prévu sur le côté ventral de la bande (8).

9. Dispositif d'amélioration de la main actionné par tendon (1) ayant un dispositif d'ancrage de force (2) selon l'une quelconque des revendications précédentes, dans lequel la bande (8) a des points de fixation (13) pour fixer un matériau d'équilibrage de force (14) sur le côté dorsal de la bande (8).
